# EUROPÄISCHE PATENTANMELDUNG

(11) **EP 1 247 528 A1**
(43) Veröffentlichungstag der Anmeldung: **09.10.2002**
(21) Anmeldenummer: 01400845.2
(22) Anmeldetag: 03.04.2001
(51) Int. Cl.: A61K 35/78, A61K 7/48, A61K 7/06, A61P 17/00

(54) **Kosmetische und/oder pharmazeutische Mittel enthaltend Extrakte aus Arachis hypogaea L**

(71) Anmelder: Cognis France S.A., 77981 Saint-Fargeau Ponthierry Cedex (FR)
(72) Erfinder: Pauly, Gilles, 54000 Nancy (FR); Henry, Florence, 54600 Villers-les-Nancy (FR); Danoux, Louis, 54420 Saulxures-les-Nancy (FR); Moser, Philippe, 54270 Essey-les-Nancy (FR)
(74) Vertreter: Cabinet HERRBURGER

(57) **Zusammenfassung**

Vorgeschlagen werden kosmetische und/oder pharmazeutische Mittel, die Extrakte aus Samenhäutchen von Arachis hypogeae L. enthalten, sowie die Verwendung dieser Extrakte in Pflegemitteln, insbesondere gegen die Hautalterung und als Proteaseinhibitoren.

## Beschreibung

### Gebiet der Erfindung

Die Erfindung liegt auf dem Gebiet der kosmetischen und/oder pharmazeutischen Mittel und betrifft Mittel, die Extrakte aus Samenhäutchen von Arachis hypogaea enthalten sowie daraus isolierte Flavonderivate und die Verwendung von Extrakten aus Samenhäutchen von Arachis hypogaea und daraus isolierte Flavonderivate in Pflegemitteln.

### Stand der Technik

Kosmetische Zubereitungen stehen dem Verbraucher heute in einer Vielzahl von Kombinationen zur Verfügung. Dennoch besteht im Markt weiterhin das Bedürfnis nach Produkten mit einem verbesserten Leistungsspektrum. Hierbei sind insbesondere Hautverträglichkeit sowie der Einsatz natürlicher Produkte beim Kunden gefragt. Daneben ist es wünschenswert, durch Kombination mit bereits bekannten Wirkstoffen, deutlich bessere Produkte zu erhalten. Von besonderem Interesse sind Stoffe, die sowohl die technischen Eigenschaften des kosmetischen Produktes, wie Lagerstabilität, Lichtstabilität und Formulierbarkeit positiv beeinflussen, als auch gleichzeitig Wirkstoffe darstellen, die beispielsweise pflegende, irritationshemmende und/oder entzündungshemmende Eigenschaften vermitteln.
Weiterhin besteht insbesonders auf dem Gebiet der sogenannten "Anti-ageing" Produkte ein dauerndes Bedürfnis nach neuen und wirkungsvolleren Stoffen, die den normalen oder krankheitsbedingten Alterungsprozess der Haut aufhalten oder verzögern können. Der Alterungsprozess der menschlichen Haut wird durch eine Vielzahl von Vorgängen ausgelöst. Dazu zählen u.a. oxidative Veränderungen in der Struktur der Haut (z.B. oxidative Degeneration von Bindegewebsproteinen, funktionelle Veränderung von Enzymen der Haut), welche durch reaktive Sauerstoffspezies oder deren Folgeprodukte in der Haut verursacht werden. Die entstehenden reaktiven Sauerstoffspezies, wie Wasserstoffperoxid oder reaktive Sauerstoff-Radikale wie Singulett-Sauerstoff, Hydroxyl, Hyperoxid-Anionen, verursachen Schäden auf Ebene der genetischen Information (DNA) sowie auf Proteinebene (Enzyme sowie strukturelle Proteine, die für die Spannkraft und die Elastizität der Haut verantwortlich sind) und auf Ebene der Lipidmembranen. Diese Schäden reichern sich über die Lebensdauer einer Zelle an und tragen so zu einer vorzeitigen Hautalterung bei.
Die komplexe Aufgabe der Erfindung hat somit darin bestanden, kosmetische Zubereitungen zur Verfügung zu stellen, die den hohen Anforderungen an Phasenstabilität und Lagerbeständigkeit sowie Verträglichkeit gegenüber empfindlicher Haut gerecht werden und möglichst zusätzlich noch deutlich verbesserte hautpflegende und schützende Eigenschaften aufweisen. Von besonderem Interesse sind hierbei Wirkstoffe, die pflegende, feuchtigkeitsspendende, irritationshemmende und/oder entzündungshemmende Eigenschaften vermitteln. Von ganz besonderem Interesse sind Wirkstoffe, welche die Hautalterung verhindern oder verlangsamen können.

### Beschreibung der Erfindung

Gegenstand der Erfindung sind kosmetische und/oder pharmazeutische Mittel, die einen Extrakt aus Samenhäutchen von Arachis hypogaea L. (Erdnuss) enthalten. Die antioxidativen Eigenschaften von Extrakten aus Samenhäutchen sind beispielsweise von G.-C. Yen und P.-D. Pu in **J. Agric. Food Chem. 1994, 42, 629-632** oder in **JAOCS, 1993, 70,383-386** beschrieben. Überraschenderweise wurde gefunden, dass Mittel, die einen Extrakt aus Samenhäutchen von Arachis hypogaea enthalten, gute hautpflegende und schützende Eigenschaften sowie gleichzeitig hohe Hautverträglichkeit aufweisen. Darüber hinaus lassen sich die Extrakte problemlos in kosmetische Formulierungen einarbeiten. Die so erhaltenen Mittel zeichnen sich weiterhin durch eine besonders hohe antioxidative Kapazität aus, die zum einen die Haut vor entzündlichen Reaktionen sowie vor oxidativ bedingten Hautalterungsvorgängen schützt, zum anderen werden gleichzeitig die Mittel vor oxidativem Abbau (Verderb) geschützt. Darüber hinaus sind die so erhaltenden Mittel stabil gegenüber mikrobiellem Befall, insbesondere gegenüber Pilzbefall, so dass in vielen Fällen auf den Zusatz weiterer Konservierungsmittel verzichtet werden kann. Dies ist insbesondere in Hinblick auf Produkte für empfindliche Haut von Vorteil, da allergische Reaktionen oder Unverträglichkeiten oft durch Konservierungsmittel ausgelöst werden.
Die Begriffe "Samenhäutchen von Arachis hypogaea" und "Erdnusshäutchen" werden im Text synonum vewendet.

### Samenhäutchen von Arachis hypogaea

Erdnüsse sind die Früchte des tropischen Schmetterlingsblütlers Arachis hypogaea, der in Indien, Westafrika, China u. Südamerika angebaut wird. Die Blütenstiele der Pflanzen krümmen sich nach dem Abblühen in den Boden und bringen dort die Erdnüsse zur Entwicklung: blaßgebliche, in der Mitte eingeschnürte, höckerige Hülsen, die 2-3 braun-rothäutige Samen enthalten. Die Erdnüsse bestehen aus einer Fruchtwand und einem von einer dünnen, rotbraunen Samenschale/Samenhäutchen umgebenen Samen (Arachidis semen, synonym: Semen Arachidis). Die erfindungsgemäßen Extrakte werden aus diesen Samenhäutchen gewonnen. Üblicherweise fallen Samenhäutchen bei der Verarbeitung von Erdnüssen an und werden manuell oder maschinell von den Erdnusssamen abgetrennt. Die so erhaltenen Samenhäutchen können zur besseren Handhabung in Form von Pellets vorliegen.

### Extraktion

Als Ausgangsmaterial können sowohl ungeröstete als auch geröstete Samenhäutchen eingesetzt werden, wie sie bei der Gewinnung von Erdnüssen anfallen. Üblicherweise werden die Samenhäutchen vor der Extraktion mechanisch zerkleinert. Hierbei eignen sich alle dem Fachmann bekannten Zerkleinerungsmethoden, als Beispiel sei die Gefriermahlung genannt.

Vor der Extraktion der Samenhäutchen kann eine Entfettung der Samenhäutchen durchgeführt werden, dies kann beispielsweise mit üblichen organischen Lösungsmitteln, wie n-Hexan, n-Heptan oder Toluol, üblicherweise bei Raumtemperatur (20 bis 25 °C, vorzugsweise 20 bis 22 °C) durchgeführt werden. Als vorteilhaft hat sich eine mehrstufige, in der Regel 3-stufige, Kreuzstromextraktion zur Entfettung erwiesen. Diese kann beispielsweise in einer Soxhlet Apparatur durchgeführt werden. Alternativ kann eine Entfettung durch CO₂-Extraktion durchgeführt werden. Durch die Entfettung können die Komponenten aus dem Rohmaterial abgetrennt, die nicht zum antioxidativen Potential des Endproduktes beitragen. Auf diese Weise können die Wirkstoffe in den Häutchen angereichert werden. Durch die vorherige Abtrennung der Fette und Öle wird darüber hinaus eine weitere Quelle des oxidativen Verderbs entfernt. Wurde eine Entfettung durchgeführt, hat es sich als vorteilhaft erwiesen, die entfetteten Samenhäutchen vor der eigentlichen Extraktion zu trocknen.

Die eigentliche Extraktion der Samenhäutchen erfolgt durch übliche Methoden der Extraktion. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei der Einfachheit halber beispielsweise auf **Hagers Handbuch der Pharmazeutischen Praxis,** (5. Auflage, Bd. 2, S. 1026-1030, Springer Verlag, Berlin-Heidelberg-New-York 1991) verwiesen. Für den großtechnischen Einsatz vorteilhaft ist die Perkolationsmethode.

Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Aceton, 1,2-Propandiol, 1,3-Propandiol, Polyethylenglykolen, Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt.

Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden.

Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion der Samenhäutchen liegen im Bereich von 3 bis 15, insbesondere 6 bis 10 Gew.-%.

Die vorliegende Erfindung umfaßt die Erkenntnis, dass die Extraktionsbedingungen sowie die Ausbeuten der Extrakte vom Fachmann je nach gewünschtem Einsatzgebiet gewählt werden können.

### Extrakte

Die erfindungsgemäß einzusetzenden Extrakte der Samenhäutchen von Arachis hypogaea L. sind je nach gewähltem Ausgangsmaterial und nach gewählter Extraktionsmethode zusammengesetzt. Die vorliegende Erfindung schließt die Erkenntnis mit ein, dass die Eigenschaften der Extrakte der Samenhäutchen durch das Zusammenwirken einer Vielzahl von - zum Teil noch nicht identifizierten
- Verbindungen zustande kommen. So sind beispielsweise die pflegenden und hautverträglichen Eigenschaften auf das Zusammenspiel der im Extrakt vorhandenen Einzelsubstanzen zurückzuführen.
   In einer besonderen Ausführungsform der Erfindung enthalten die Extrakte Flavonderivate, die ausgewählt sind aus der Gruppe, die gebildet wird von Luteolin, procyanidolischen Oligomeren, Procyanidin B1, Proanthocyanidinen, Proanthioanthocyanidin A2. Bevorzugt enthalten die Extrakte aus Samenhäutchen von Arachis hypogaea L. als Hauptkomponente (> 25 Gew.-% bezogen auf die Menge des Gesamtextraktes) Luteolin. Besonders bevorzugt ist der Einsatz eines Extraktes aus Samenhäutchen von Arachis hypogaea L., der über 40 Gew.-%, insbesondere über 50 Gew.-% Luteolin enthält.

### Luteolin

Luteolin gehört zu den Flavonderivaten, es handelt sich um ein vierfach substituiertes Flavon: 3',4'5,7-Tetrahydroxyflavon (C₁₅H₁₀O₆). Neben seinem Vorkommen in den Samenhäutchen von Arachis hypogaea ist es in Blättern, Blüten und Stengeln des Färberwau (Färberreseda, Gelbkraut, Reseda luteola) zu finden, des weiteren in den Blüten des gelben Fingerhuts (Digitalis lutea).

### Einsatzmenge

In der Regel enthalten die Mittel 0,01 bis 20 Gew.-%, vorzugsweise 0,05 bis 5, und insbesondere 0,1 bis 2,5 Gew. % des Extraktes aus Samenhäutchen berechnet als Trockensubstanz, bezogen auf das Mittel, mit der Massgabe, dass sich die Mengenangaben mit Wasser und gegebenenfalls weiteren Hilfs- und Zusatzstoffen zu 100 Gew.-% addieren.

Ein weiterer Gegenstand der Erfindung ist die Verwendung von Extrakten aus Samenhäutchen von Arachis hypogaea als Pflegemittel für Haut und/oder Haar. Bevorzugt sind hierbei Flavonderivathaltige Extrakte und besonders bevorzugt Luteolin-haltige und Procyanidin B1-haltige Extrakte. Die Art der Verwendung umfasst sowohl Mittel mit kosmetischer als auch mit pharmazeutischer Wirkung.

### Pflegemittel:

Als Pflegemittel im Sinne der Erfindung sind Pflegemittel für Haut und Haar zu verstehen. Diese Pflegemittel schließen unter anderem reinigende und aufbauende Wirkung für Haut und Haare ein.

Die Applikation kann sowohl topisch als auch oral in Form von Tabletten, Dragees, Kapseln, Säfte, Lösungen und Granulate erfolgen.

Die erfindungsgemäßen Zubereitungen zeigen eine hervorragende hautpflegende Wirkung bei gleichzeitig hoher Hautverträglichkeit. Die Zubereitungen weisen eine Vielzahl von kosmetischen und pharmazeutischen Wirkungen auf. Weitere Gegenstände der Erfindung betreffen daher die Verwendung von Extrakten aus Samenhäutchen von Arachis hypogaea bevorzugt Flavonderivathaltige Extrakte
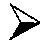 als Antioxidans;
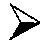 gegen freie Radikale;
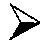 als Sonnenschutzmittel; insbesondere gegen UVA-Strahlung und/oder gegen UVB-Strahlung;
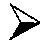 als anti-inflammatorische Mittel
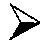 als Mittel gegen die Hautalterung
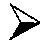 als protease-inhibierendes Mittel, insbesondere als MMP- und/oder Collagenase- und/oder Elastase-inhibierendes Mittel;
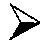 als Stimulator der TIMP's.

Überraschenderweise wurde gefunden, dass der Extrakt aus Samenhäutchen nicht nur an sich antioxidativ wirkt, sondern sich auch als antioxidativer Wirkstoff für kosmetische Mittel eignet. Dabei werden nicht nur die kosmetischen Mittel selbst vor oxidativer Zersetzung geschützt, sondern die durch Oxidation bedingten Veränderungen der Haut wirkungsvoll verhindert oder zumindest verzögert. Die erfindungsgemäßen Extrakte zeigen darüber hinaus eine hervorragende hautpflegende Wirkung bei gleichzeitig hoher Hautverträglichkeit. Darüber bestehen hinsichtlich der Einarbeitbarkeit in kosmetische Formulierungen sowie der Stabilität der erhaltenen Produkte keine Einschränkungen.

Als Antioxidantien im Sinne der Erfindung sind Oxidationsinhibitoren zu verstehen, die sich aus Samenhäutchen von Arachis hypogaea isolieren lassen. Antioxidantien sind in der Lage, die unerwünschten, durch Sauerstoff-Einwirkungen und andere oxidative Prozesse bedingten Veränderungen in den zu schützenden Stoffen zu hemmen oder zu verhindern. Die Wirkung der Antioxidantien besteht meist darin, dass sie als Radikalfänger für die bei der Autoxidation auftretenden freien Radikale wirken.

Neben der Verwendung von Extrakten der Samenhäutchen von Arachis hypogaea als Antioxidans können auch weitere, bereits bekannte Antioxidantien eingesetzt werden. Eine mögliche Anwendung der Antioxidantien zum Beispiel in kosmetischen und/oder pharmazeutischen Mitteln, ist die Anwendung als sekundäre Lichtschutzmittel, weil Antioxidantien in der Lage sind, die photochemische Reaktionskette zu unterbrechen, welche ausgelöst wird, wenn UV-Strahlung in die Haut eindringt. Neben dem erfindungsgemäßen Pflanzenextrakt sind weitere typische Beispiele hierfür Aminosäuren (z.B. Glycin, Alanin, Arginin, Serin, Threonin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin, Lutein) oder deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, Boldin, Boldo-Extrakt, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-Eacetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Neben der Verwendung als Antioxidans und gegen freie Radikale ist es auch möglich, die Extrakte aus Samenhäutchen von Arachis hypogaea als hautaufhellendes Mittel bzw. skin-whitener einzusetzen, da die Bildung von Melanin durch den Einsatz der erfindugnsgemäßen Extrakte verringert werden kann.

### Sonnenschutzmittel bzw. UV-Lichtschutzfaktoren

Die Extrakte aus Samenhäutchen von Arachis hypogaea wirken im Sinne der Erfindung als Sonnenschutzmittel, insbesondere gegne UVA- und/oder UVB-Strahlung. Bevorzugt sind hierbei die Extrakte, die Flavonderivate enthalten.

Als Sonnenschutzmittel bzw. UV-Lichtschutzfaktoren im Sinne der Erfindung werden Lichtschutzmittel bezeichnet, die für den Schutz der menschlichen Haut gegenüber schädigenden Einflüssen der direkten und indirekten Strahlung der Sonne nützlich sind. Die für die Hautbräunung verantwortliche Ultraviolettstrahlung der Sonne unterteilt man in die Abschnitte UV-C (Wellenlängen 200-280 nm), UV-B (280-315 nm) u. UV-A (315-400 nm).

Die Pigmentierung normaler Haut unter dem Einfluss der Sonnenstrahlung, d. h. die Bildung von Melaninen, wird durch UV-B u. UV-A unterschiedlich bewirkt. Bestrahlung mit UV-A-Strahlen ("langwelligem UV") hat die Dunkelung der in der Epidermis bereits vorhandenen Melanin-Körper zur Folge, ohne dass schädigende Einflüsse zu erkennen sind. Anders bei dem sog. "kurzwelligen UV" (UV-B). Dieses bewirkt die Entstehung von sog. Spätpigment durch Neubildung von Melanin-Körnern. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) u, gar Brandblasen führen kann.

Als UV-Absorber oder Lichtfilter, die also die UV-Strahlung in unschädliche Wärme umwandeln, werden Extrakte aus der Fruchthülle der Samenhäutchen von Arachis hypogaea eingesetzt, diese können zusätzlich in Kombination mit weiteren Sonnenschutzmitteln bzw. UV-Lichtschutzfaktoren vorliegen.

Diese weiteren UV- Lichtschutzfaktoren sind beispielsweise bei Raumtemperatur flüssig oder kristallin vorliegende organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
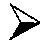 3-Benzylidencampher bzw. 3-Benzylidennorcampher und dessen Derivate, z.B. 3-(4-Methylbenzyliden)campher wie in der **EP 0693471 B1** beschrieben;
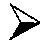 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
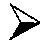 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octo-crylene);
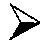 Ester der Salicylsäure, vorzugsweise Salicylsäure-2-ethylhexylester, Salicylsäure-4-isopropylbenzylester, Salicylsäurehomomenthylester;
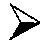 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
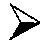 Ester der Benzalmalonsäure, vorzugsweise 4-Methoxybenzmalonsäuredi-2-ethylhexylester;
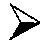 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
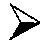 Propan-1,3-dione, wie z.B. 1-(4-tert.Butylphenyl)-3-(4'methoxyphenyl)propan-1,3-dion;
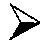 Ketotricyclo(5.2.1.0)decan-Derivate, wie in der **EP 0694521 B1** beschrieben. Als wasserlösliche Substanzen kommen in Frage:
2-Phenylbenzimidazol-5-sulfonsäure und deren Alkali-, Erdalkali-, Ammonium-, Alkylammonium-, Alkanolammonium- und Glucammoniumsalze;
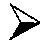 Sulfonsäurederivate von Benzophenonen, vorzugsweise 2-Hydroxy-4-methoxybenzophenon-5-sulfonsäure und ihre Salze;
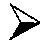 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UVA-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol 1789), 1-Phenyl-3-(4'-isopropylphenyl)-propan-1,3-dion sowie Enaminverbindungen, wie beispielsweise beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Dimethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parfümerie und Kosmetik 3 (1999), Seite 11ff** zu entnehmen.

Die Extrakte aus Samenhäutchen von Arachis hypogaea wirken im Sinne der Erfindung gegen die Schädigung von Fibroblasten und/oder Keratinocyten durch UVA-Strahlung und/oder UVB-Strahlung.

UVA-Strahlen dringen bis in die Dermis ein, wo sie zu Oxidationsstreß führen, was durch eine Lipoperoxidation der Zytoplasmamembranen nachgewiesen wird. Die Lipoperoxide werden zu Malonaldialdehyd (MDA) abgebaut, der viele biologische Moleküle wie Proteine und Nukleinbasen vernetzen wird (Enzymhemmung bzw. Mutagenese). Die erfindungsgemäßen Extrakte aus Samenhäutchen von Arachis hypogaea reduzieren signifikant den Grad an MDA in humanen Fibroblasten, welcher durch UVA-Strahlen induziert wird und zeigen damit eine hohe Kapazität schädliche Effekte eines oxidativen Stresses auf der Haut zu reduzieren.

UVB-Strahlen lösen durch Aktivierung eines Enzyms, nämlich Phospholipase A2 oder PLA2 eine Entzündung aus. Diese Entzündung (Erythem, Ödem) wird durch die Entfernung von Arachidonsäure aus den Phospholipiden der Plasmamembran durch die Phospholipase ausgelöst. Arachidonsäure ist die Vorstufe der Prostaglandine, die eine Entzündung und eine Zellmembranschädigung verursachen; die Prostaglandine E2 (= PGE2) werden durch die Cyclooxygenase gebildet. Der Grad der Freisetzung des Cytoplasaenzyms LDH (Lactat Dehydrogenase) in humanen Keratinocyten dient als Marker für eine Zellschädigung.

Die erfindungsgemäßen Extrakte aus Samenhäutchen von Arachis hypogaea reduzieren den Effekt von UVB-Strahlung auf die Anzahl an Keratinocyten und auf den Gehalt an freigesetzte LDH. Die Extrakte zeigen demnach die Fähigkeit, die durch UVB-Strahlung hervorgerufene Schädigung an Zellmembranen zu reduzieren.

Die Extrakte aus Samenhäutchen von Arachis hypogaea bevorzugt die Flavonderivat-haltigen Extrakte wirken im Sinne der Erfindung als Antioxidans bzw gegen frei Radikale. Die weiteren UV-Lichtschutzfaktoren bzw. Antioxidantien können in Mengen von 0,01 bis 25, vorzugsweise 0,03 bis 10 und insbesondere 0,1 bis 5 Gew.-% bezogen auf die Gesamtmenge in den Zubereitungen, zugegeben werden.

Die Extrakte aus Samenhäutchen von Arachis hypogaea, bevorzugt die Flavonderivat-haltigen Extrakte wirken im Sinne der Erfindung als anti-inflammatorisches Pflegemittel, die eine Entzündung der Haut heilen können oder die einer Entzündung vorbeugen können. Insbesondere wirken die erfindungsgemäßen Mittel als anti-rosacea Mittel. Die Entzündungen können weiterhin die unterschiedlichsten Ursachen aufweisen. Insbesondere können Entzündungen behandelt werden, die durch UV-Strahlung, Hautverunreinigungen oder bakteriell wie hormonell bedingte Hautveränderungen, z. B. Akne induziert werden.

Die Extrakte aus Samenhäutchen von Arachis hypogaea, bevorzugt die Flavonderivat-haltigen Extrakte wirken im Sinne der Erfindung gegen Hautalterungen, insbesondere gegen jede Art der Fältchen- und Faltenbildung. Eine andere Bezeichnung für diese Art der Pflegemittel ist auch antiageing Mittel. Die Verwendungen schließen eine Verlangsamung von Altersprozessen der Haut mit ein. Die Alterserscheinungen können die unterschiedlichsten Ursachen aufweisen. Insbesondere können diese Alterserscheinungen auf Grund von Apoptose, durch UV-Strahlung oder durch die Zerstörung der hauteigenen Proteine wie beispielsweise Collagen oder Elastan induzierten Schädigungen der Haut verursacht sein. Die erfindungsgemäßen Extrakte aus Samenhäutchen von Arachis hypogaea wirken als Protease-inhibierendes Mittel, insbesondere als MMP- und/oder Collagenase- und/oder Elastase-inhibierendes Mittel. Unter MMP versteht man Matrix-Metallo-Proteasen. Zu den Matrix-Metallo-Proteasen zählen u.a. Collagenase, aber auch eine bestimmte Art der Elastasen. Die Aktivität der Enzyme ist abhängig von Metallionen - häufig handelt es sich um Zn²⁺-Ionen. Die hauptsächlich vorkommende Elastase zählt zur Gruppe der Serin-Proteasen. Ihre katalytische Reaktion beruht auf einen anderen Mechanismus. Diese Proteasen (Collagenase und die unterschiedlichen Elastasen) katalysieren die Fragmentierung und Zerstörung der dermalen Makromoleküle wie Proteoglycan, Collagen und Elastin und führen dadurch zur Alterung der Haut und zu den Effekten der natürlichen Hautalterung nach UV-Strahlung.

Bei inflammatorischen Prozessen in der Haut werden von den Makrophagen und von polymorphonuclearen neutrophilen Granulocyten Proteasen wie z.B. die Serin-Protease Elastase oder Matrix-Metallo-Proteasen (MMP) wie Collagenase und eine weitere, zu den MMP gehörende Elastin abbauende Elastase ausgeschüttet. Des weiteren werden bei älteren Menschen oder nach UV-Strahlung durch die dermalen Fibroblasten interstitial Collagenasen - oder auch MMP-1 genannt - ausgeschüttet.

Im menschlichen Gewebe finden sich Inhibitoren dieser Matrix-Metallo-Proteasen, deren Bildung und Konzentration mit dem Alter abnimmt. Ihre Bezeichnung wird mit TIMP (Tissue-inhibitor of metallo-protease) abgekürzt. Die erfindungsgemäßen Extrakte sind in der Lage die Bildung dieser natürlich vorkommenden Inhibitoren zu stimulieren. Ein weiterer Gegenstand der Erfindung ist die Verwendung von Extrakten aus Samenhäutchen von Arachis hypogaea als Stimulatoren der Tissue-inhibitor of Metallo-protease.

Die Verwendung der erfindungsgemäßen Extrakte als schützende und aufbauende Pflegemittel ist prinzipiell für alle Zubereitungen möglich, die zur Prävention gegen Schädigungen oder bei Schädigungen der Haut und/oder Haare und damit in der Haut- und Haarpflege eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

### Kosmetische Mittel

Die erfindungsgemäßen Extrakte können zur Herstellung von kosmetischen Mitteln, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, Hydrotrope, Konservierungsmittel, Insektenrepellentien, Selbstbräuner, Tyrosininhibitoren (Depigmentierungsmittel), Solubilisatoren, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
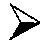 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
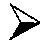 Alkyl- und/oder Alkenyloligoglykoside mit 8 bis 22 Kohlenstoffatomen im Alk(en)ylrest und deren ethoxylierte Analoga;
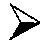 Anlagerungsprodukte von 1 bis 15 Mol Ethylenoxid an Ricinusöl und/odergehärtetes Ricinusöl;
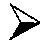 Anlagerungsprodukte von 15 bis 60 Mol Ethylenoxid an Ricinusöl und/oder gehärtetes Ricinusöl;
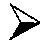 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
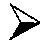 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
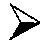 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
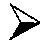 Mono-, Di- und Trialkylphosphate sowie Mono-, Di- und/oder Tri-PEG-alkylphosphate und deren Salze;
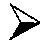 Wollwachsalkohole;
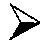 Polysiloxan-Polyalkyl-Polyether-Copolymere bzw. entsprechende Derivate;
Polyalkylenglycole sowie
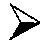 Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside,** ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI),

Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und-diester von Fettsäuren, Polyacrylate, (z.B. Carbopole® von Goodrich oder Synthalene® von Sigma), Poly-acrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxy-propyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, un-vernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxyproyl-methacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Carnaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Als **biogene Wirkstoffe** können eingesetzt werden (Desoxy)Ribonucleinsäuren, pflanzliche Wirkstoffe und/oder Vitamine.

Unter **(Desoxy)Ribonucleinsäuren** (DNA bzw. RNA) werden hochmolekulare, fadenförmige Polynuc-leotide verstanden, die sich von 2'-Desoxy-β-D-ribonucleosiden bzw. D-Ribonucleosiden ableiten, die ihrerseits wieder von äquivalenten Mengen einer Nucleobase und der Pentose 2-Desoxy-D-ribo-furanose bzw. D-Ribofuranose aufgebaut werden. Der Einsatz von Nucleinsäuren als Wirkstoffe in der Kosmetik ist bekannt. So werden beispielsweise in der französischen Patentanmeldung **FR-A1 2511253** Haut- und Sonnenschutzmittel mit einem Gehalt an hochpolymerisierter DNA vorgeschlagen. Aus der japanischen Offenlegungsschrift **JP-A2 62/096404** (Kanebo) sind kosmetische Zusammen-setzungen mit Nucleinsäuren und Diisopropylamindichloracetat bekannt. Gegenstand der franzö-sischen Patentschrift **FR-B1 2620024** (Soc.d'Etudes Dermatologiques) sind Zubereitungen, enthaltend Nucleinsäurederivate als Radikalfänger. Beispiele sind Adenin, Guanosin, Xanthin, Hypoxanthin, Uracil und Ribonucleinsäure. In der internationalen Patentanmeldung **WO 95/01773** (Boston University) wird ein Verfahren zur Stimulation der Pigmentproduktion beschrieben, bei dem man DNA-Fragmente, bevorzugt Dinucleotide, in liposomaler Form in die Epidermis transportiert. Gegenstand der deutschen Patentanmeldung **DE-A1 4323615** sind schließlich Zusammensetzungen mit einem Gehalt an Nucleinsäuren und deren Fragmenten als Anti-Ageing- und Sonnenschutzcremes.

Als Nucleobasen können die DNA bzw. RNA die Purinderivate Adenin und Guanin sowie die Pyrimidine Cytosin und Thymin bzw. Uracil enthalten. In den Nucleinsäuren sind die Nucleobasen N-glykosidisch mit Kohlenstoffatom 1 der Ribose, wodurch im Einzelfall Adenosine, Guanosine, Cytidine und Thimidine entstehen. In den Säuren verknüpft eine Phosphatgruppe die 5'-Hydroxygruppe der Nucleoside mit der 3'-OH-Gruppe der jeweils folgenden durch eine Phosphodiesterbrücke unter Ausbildung von Einzel-strang-DNA bzw. -RNA. In der Regel liegt die DNA doppelsträngig vor (Ausbildung von Wasserstoffbrückenbindungen zwischen den entsprechenden Basen) und die RNA einzelsträngig. Je nach Behandlung der Nucleinsäuren, können sowohl DNA als auch RNA in Form von Doppelsträngen und/oder Einzelsträngen vorliegen. Auch Heterodimere (Doppelstrang zwischen DNA und RNA) sind möglich. Unter dem Begriff (Desoxy)Ribonucleinsäuren im Sinne der vorliegenden Erfindung werden sowohl doppel- als auch einzelsträngige (Desoxy)Ribonucleinsäuren verstanden, des weiteren Mischungen aus Einzelsträngen und Doppelsträngen. Wegen des großen Verhältnisses von Länge zu Durchmesser neigen DNA- bzw. RNA-Moleküle schon bei mechanischer Beanspruchung, etwa bei der Extraktion, zu Strangbruch. Aus diesem Grunde kann das Molekulargewicht der Nucleinsäuren 10³ bis 10⁹ Dalton erreichen. Im Sinne der Erfindung werden konzentrierte DNA bzw. RNA-Lösungen eingesetzt, die sich durch ein flüssig-kristallines Verhalten auszeichnen. Vorzugsweise werden Desoxy- bzw. Ribonucleinsäuren eingesetzt, die aus marinen Quellen beispielsweise durch Extraktion von Fischsperma erhalten werden und die ein Molekulargewicht im Bereich von 1000 bis 5.000.000 Dalton aufweisen. Besonders bevorzugt ist der Einsatz von einzelsträngigen Desoxyribonucleinsäuren marinen Ursprungs mit einem Molekulargewicht im Bereich von 10.000 bis 100.000 Dalton. Die (Desoxy)Ribonucleinsäuren können in den erfindungsgemäßen Mitteln in

Mengen von 0,001 bis 2,5, vorzugsweise 0,1 bis 0,5 Gew.-% - bezogen auf das Mittel - enthalten sein.

Eine Vielzahl von **pflanzlichen Wirkstoffen** hat beruhigende, pflegende, feuchtigkeitsspendende, zum Teil auch entzündungshemmende, UV-absorbierende, hautaufhellende und/oder selbstbräunende Wirkung auf die Haut. Aus diesem Grund ist der Einsatz dieser Stoffe in kosmetischen und/oder pharmazeutischen Zubereitungen von großer Bedeutung. Beispiele für pflanzliche Stoffe mit diesem Leistungsspektrum neben dem erfindungsgemäßen Extrakt sind Allantoin, Bisabolol, Panthenol, Ferulasäure (4-Hydroxy-3-methoxyzimtsäure) sowie Phytosterole. Der Einsatz dieser Mittel beträgt in der Regel 0,001 - 3 Gew.-% Aktivsubstanz bezogen auf das kosmetische Mittel. Weitere mögliche pflanzliche Wirkstoffe, die den erfindungsgemäßen Mitteln zugesetzt werden können sind Phenole sowie Polyphenole.

Den erfindungsgemäßen Mitteln können weiterhin **Vitamine** enthalten, wie beispielsweise Tocopherole, Ascorbinsäure, Carotinoide, Biotin und Vitamin A.

Unter Tocopherolen versteht man in 2-Stellung mit einem 4,8,12,-Trimethyltridecyl-Rest substituierte Chroman-6-ole (3,4-Dihydro-2H-benzopyran-6-ole). In die gleiche Gruppe der Biochinone, d.h. zu den polyprenylierten 1,4-Benzo- bzw. Naphthochinonen, gehören die Plastochinone, Tocopherolchinone, Ubichinone, Bovichinone, K-Vitamine, Menachinone (2-Methyl-1,4-naphthochinone). In Frage kommen insbesondere α-, β-, γ- und d-Tocopherole die der allgemeinen Formel **(I)** folgen (R = Wasserstoff oder Methyl), die ε-Tocopherole der allgemeinen Formel **(II),** die noch über die ursprüngliche ungesättigte Prenylseitenkette verfügen, sowie die α-Tocopherolchinone und -hydrochinone der allgemeinen Formel **(III),** bei denen das Pyran-Ringsystem geöffnet ist.

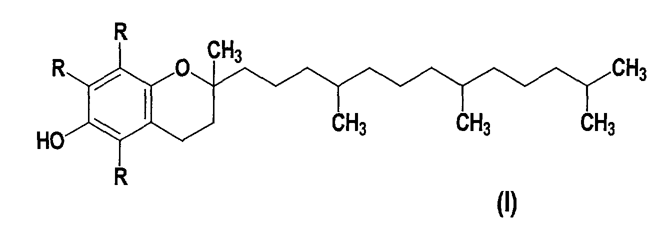

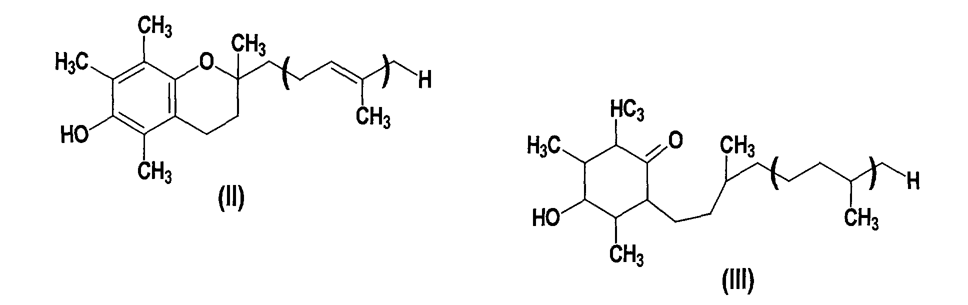

Neben den Tocopherolen kommen auch deren Derivate, insbesondere Ester mit Carbonsäuren, wie beispielsweise Tocopherolacetat oder -palmitat in Frage. Die Tocopherole können in den erfindungsgemäßen Mitteln in Mengen von 0,001 bis 20,0 Gew.-%, vorzugsweise von 0,2 bis 2,5 Gew.-% - bezogen auf das Mittel - enthalten sein.

Ascorbinsäure (Vitamin C) wird als wasserlösliches Vitamin in einer Vielzahl von kosmetischen Produkten eingesetzt. Hier dient es in der Regel als natürliches Antioxidans, oft in Kombination mit fettlöslichen Vitaminen wie Tocopherolen. Ascorbinsäure wird in der Regel als freie Säure oder in Form von Ascorbylpalmitat oder Ascorbylacetat eingesetzt. In der Regel ist Ascorbinsäure in den erfindungsgemäßen Mitteln in Mengen von 0,05 bis 1,0 Gew.-%, vorzugsweise von 0,1 bis 0,5 Gew.-% - bezogen auf das Mittel - enthalten.

Die Carotinoide an sich haben keinen Vitamincharakter, sie werden aufgrund der Funktion der β-Carotins als Provitamin A jedoch im Sinne dieser Anmeldung unter den Begriff der Vitamine miterfaßt.

Unter Carotinoiden werden von Carotin abgeleitete, kohlenstoffhaltige Substanzen zusammengefaßt, deren Grundgerüst aus acht Isopren-Einheiten (Tetraterpen) besteht. Die Sauerstoffhaltigen Derivate der Carotinoide werden als Xanthophylle bezeichnet. Die 3 bedeutendsten Isomere des Carotins sind: α-, β- und γ- Carotin. Alle 3 besitzen das gleiche Grundgerüst mit 9 konjugierten Doppelbindungen, 8 Methyl-Verzweigungen und einer β-lonon Struktur am Molekülende. β-Carotin ist das in der Natur am häufigsten vorkommende Carotinoid. α-Carotin leite sich formal vom β-Carotin durch Verschiebung der Doppelbindung in die 4',5'-Stellung ab, im γ-Carotin ist der rechte Ring zwischen C-1'und C-6'geöffnet, wodurch eine neue Doppelbindung entsteht. Weitere Carotinoide sind δ-, ξ-, und ε- Carotin. Das wichtigste Carotin Isomer ist β-Carotin, das eigentliche Provitamin A, das im tierischen Organismus in zwei Moleküle Retinal gespalten wird. Carotinoide können in den erfindungsgemäßen Mitteln in Mengen von 0,05 bis 1,0 Gew.-%, vorzugsweise von 0,1 bis 0,5 Gew.-% - bezogen auf das Mittel -enthalten sein.

Biotin (D-cis-Hexahydro-2-oxothienol[3,4-*d*]imidazol-4-valeriansäure), gehört zu den Vitaminen der B Gruppe (veraltete Bezeichnung für Biotin ist Vitamin H). Biotin kann in den erfindungsgemäßen Mitteln in Mengen von 0,001 bis 2,5 Gew.-%, insbesondere in Mengen von 0,01 bis 1,0 Gew.-%, bezogen auf das Mittel-, enthalten sein.

Unter Vitamin A im Sinne der vorliegenden Erfindung werden Retinol, Retinal (synonym Retinaldehyd) und Retinsäure sowie deren Stereoisomere und Ester zusammengefaßt, aber auch alle Verbindungen, die sich von der Stammverbindung Retinol (all-trans Retinol) ableiten, auch synthetisch hergestellte, die nicht in der Natur vorkommen (sog. Retinoide). Vitamin A kann in den erfindungsgemäßen Mitteln in Mengen von 0,001 bis 0,01 Gew.-% -bezogen auf das Mittelenthalten sein.

Die Gesamtmenge an Vitaminen im Endprodukt liegt in der Regel zwischen 0,001 bis 25 Gew.-%-bezogen auf das Mittel-, vorzugsweise 0,01 bis 5 Gew.-% . Besonders bevorzugt ist ein Einsatz von 0,1 bis 2,5 Gew.-%.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als weitere **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
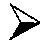 adstringierende Wirkstoffe,
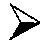 Ölkomponenten,
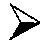 nichtionische Emulgatoren,
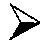 Coemulgatoren,
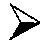 Konsistenzgeber,
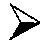 Hilfsstoffe wie z. B. Verdicker oder Komplexierungsmittel und/oder
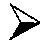 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

Daneben können in Antitranspirantien übliche öllösliche und wasserlösliche Hilfsmittel in geringeren Mengen enthalten sein. Solche öllöslichen Hilfsmittel können z.B. sein:
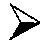 entzündungshemmende, hautschützende oder wohlriechende ätherische Öle,
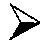 synthetische hautschützende Wirkstoffe und/oder
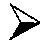 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Octopirox® (1-Hydroxy-4-methyl-6-(2,4,4-trimythylpentyl)-2-(1H)-pyridon-monoethanolaminsalz), Baypival, Pirocton Olamin, Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Selendisulfid, Schwefel kolloidal, Schwefelpolyehtylenglykolsorbitanmonooleat, Schwefelrizinolpolyehtoxylat, Schwe-felteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat, Zinkpyrethion, Aluminiumpyrition und Magnesiumpyrithion / Dipyrithion-Magnesiomsulfat eingesetzt werden.

Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
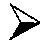 Glycerin;
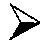 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
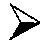 technische Oligoglyceringemische mit einem Eigenkondensationsgrad von 1,5 bis 10 wie etwa technische Diglyceringemische mit einem Diglyceringehalt von 40 bis 50 Gew.-%;
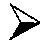 Methyolverbindungen, wie insbesondere Trimethylolethan, Trimethylolpropan, Trimethylolbutan, Pentaerythrit und Dipentaerythrit;
Niedrigalkylglucoside, insbesondere solche mit 1 bis 8 Kohlenstoffen im Alkylrest, wie beispielsweise Methyl- und Butylglucosid;
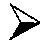 Zuckeralkohole mit 5 bis 12 Kohlenstoffatomen, wie beispielsweise Sorbit oder Mannit,
Zucker mit 5 bis 12 Kohlenstoffatomen, wie beispielsweise Glucose oder Saccharose;
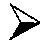 Aminozucker, wie beispielsweise Glucamin;
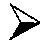 Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton. Als **Tyrosinhinbitoren,** die die Bildung von Melanin verhindern und Anwendung in Depigmentie-rungsmitteln finden, kommen beispielsweise Arbutin, Kojisäure, Cumarinsäure und Ascorbinsäure (Vitamin C) in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984,** S.81-106 zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-%bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

### 1. Herstellung der Extrakte

### Beispiel 1 - mit vorheriger Entfettung

143g Erdnusshäutchen wurden in einer Laborstiftmühle mit einem 2 mm-Siebeinsatz gefriergemahlen. In einer ersten Extraktionsreihe wurde das Erdnussöl aus den Erdnusshäutchen entfernt, dies geschah mittels einer dreistufigen Kreuzstromextraktion mit n-Hexan: hierzu wurde das Mahlgut mit 143 g n-Hexan 6 Stunden bei Raumtemperatur (20-22°C) extrahiert. Nach der Extraktion erfolgte eine Filtration, wobei der Filtrationsrückstand erneut mit 143 g frischen n-Hexan 6 Stunden bei 20 °C extrahiert wurde. Dieser Vorhang wurde noch einmal wiederholt, so dass insgesamt 3 Extraktion ä 6 Stunden durchgeführt wurden. Der Extraktionsrückstand wurde bei 30 °C im Vakuum (10 mbar) getrocknet. Der getrocknete Extraktionsrückstand (102 g) wurde dann mittels einer dreistufigen Kreuzstromextraktion mit Ethanol ebenfalls bei Raumtemperatur (20-22°C) extrahiert: hierzu wurde das getrocknete Material mit 203 g Ethanol versetzt und 6 Stunden bei 20 °C extrahiert. Die Extraktionslösung wurde filtriert, das Filtrat wurde gesammelt und der Filtrationsrückstand erneut mit 203 g Ethanol versetzt und 6 Stunden bei 20 °C extrahiert. Die Extraktionslösung wurde filtriert, das Filtrat wurde gesammelt und er Filtrationsrückstand wurde erneut mit 203 g Ethanol versetzt und 6 Stunden bei 20 °C extrahiert. Die insgesamt 3 Filtrate wurde vereinigt und bei 44 °C und 100 mbar destilliert zur Abtrennung des Ethanols. Es wurden 12,5 g eines rotbraunen Pulvers erhalten. Daraus berechnet sich eine Ausbeute von 8,7 Gew.-% bezogen auf den eingesetzten Rohstoff.

### Beispiel 2 - ohne vorherige Entfettung

139 g Erdnusshäutchen wurde in einer Laborstiftmühle mit einem 2 mm-Siebeinsatz gefriergemahlen. Das Mahlgut wurde durch dreistufige Kreuzstromextraktion mit Ethanol extrahiert: hierzu wurde das Mahlgut dreimal mit jeweils 227 g frischem Ethanol für jeweils 3,5 Stunden bei 20 °C extrahiert. Jeder Extraktionsstufe folgte eine Filtration, wobei der Filtrationsrückstand jeweils in der nächsten Extraktion eingesetzt wurde. Nach der dritten Extraktion und Filtration wurden die Filtrate vereinigt und der Ethanol abgezogen, wobei 31,3 g einer rotbraunen, fetthaltigen Masse erhalten wurden.

### Beispiel 3 - Extraktion mit 1,2 Propandiol

100 g Erdnusshäutchen wurden in einer Laborstiftmühle mit einem 2 mm Siebeinsatz gefriergemahlen. Das Mahlgut wurde mit 200 g 1,2 Propandiol 3 h bei 52 °C gerührt. Danach wurde die Mischung filtriert, das Filtrat gesammelt und der Filtrationsrückstand wurde erneut mit 200g 1,2 Propandiol 3 h bei 52 °C gerührt. Die Mischung wurde filtriert und das Filtrat mit dem Filtrat aus der ersten Extraktion vereinigt. Man erhielt eine dunkelbraune, klare Lösung mit einem Trockensubstanzgehalt von 13 g, das entspricht einer Ausbeute von 13,0 Gew.-% bezogen auf das eingesetzte Mahlgut.

### Beispiel 4 - großtechnischer Ansatz

10 kg Erdnusshäutchen wurden mit n-Hexan nach dem Soxhlet-Prinzip zunächst mit n-Hexan entfettet: dazu wurde die Erdnusshäutchenschicht (Festbett) mit einem n-Hexan Strom von 10 kg/h 4,8 Stunden bei 60 °C durchströmt. Zur Aufkonzentrierung der den Extraktor verlassenden Extraktlösung wurde ein Rücklaufstrom von 3,3 kg/h eingestellt. Der Lösemitteldurchsatz betrug somit 42 kg (Lösemittelverhältnis LMV = 4,2). Der entfettete Extraktionsrückstand wurde bei 90 °C und 10 mbar getrocknet. Anschließend wurde der entfettete und getrocknete Extraktionsrückstand (8,1 kg) mit Ethanol in einer Miniplant Anlage nach dem Soxhlet-Prinzip extrahiert. Dazu wurde das Festbett 8 Stunden lang bei 73 °C von einem Lösungsmittelstrom (Ethanol) von 8,5 kg/h durchströmt. Der Rücklaufstrom betrug 3,9 kg/h. Bei einem Durchsatz von 68 kg Ethanol ergab sich ein Lösemittelverhältnis von 8,5. Aus dem so erhaltenen ethanolischen Extrakt wurde bei 120 °C und 10 mbar das Lösungsmittel abgetrennt und es wurden 995 g eines rotbraunen Pulvers erhalten. Dies entspricht einer Ausbeute von 9,95 Gew.-% bezogen auf das eingesetzte Ausgangsmaterial.

### Beispiel 5 Entfettung mit CO₂

500 g Erdnusshäutchen wurden zum Zwecke der Entfettung mit CO₂ bei 300 bar und 60 °C erschöpfend extrahiert. Der Extraktor der verwendeten Hochdruckanlage hatte ein Volumen von ca. 1,4 Litern bei einem Innendurchmesser von 7 cm. Das relativ große Extraktorvolumen bewirkt eine nahezu pulsationsfreie Extraktion. Die Durchströmung des Materials erfolgte von unten nach oben, bei einem CO2-Strom von 2,9 kg/h. Durch zweistufige Abscheidung des Extraktes wurde eine zusätzliche Separation des gelösten Wassers ermöglicht. Die erste Abscheidung erfolgte bei 75 °C und bei 50 bis 55 bar (Abscheidung von Öl), die 2. Abscheidung erfolgte bei 20 bis 22 °C bei 50 bis 55 bar (Abscheidung von wässerigen Fraktionen). Die Extraktion wurde durchgeführt, bis keine nennenswerten Ölmengen mehr extrahiert wurden (ungefähr nach 6 Stunden). Die Ausbeute an wässeriger Fraktion betrug 9,77 g, das entspricht 1,95 Gew.% bezogen auf das eingesetzte Ausgangsmaterial. 100 g des Raffinats aus der CO₂-Extraktion wurden mit Ethanol in einer Soxhlet-Apparatur 4 Stunden bei 78 °C extrahiert. Die rotbraune Extraktionslösung wurde anschließend bei 50 °C und 20 mbar eingedampft. Man erhielt 8,5 g eines rotbraunen Pulvers, entsprechend einer Ausbeute von 7,3 Gew.% bezogen auf die eingesetzte Ausgangsmenge.

Beispiel 6 Extraktion verschieden gerösteter Erdnusshäutchen

Erdnusshäutchen unterschiedlicher Herkunft und nach bei verschiedenen Temperaturen geröstet wurden extrahiert

### Beispiel 6a)

81,4 g Erdnusshäutchen (Herkunft USA), die einer 4fachen Röstung bei jeweils 85 °C unterzogen wurden, wurden in einer Laborstiftmühle mit einem 2 mm Siebeinsatz gefriergemahlen. In einer ersten Extraktionsreihe wurde das Erdnussöl aus den Erdnusshäutchen entfernt, dies geschah mittels einer dreistufigen Kreuzstromextraktion mit n-Hexan: hierzu wurde das Mahlgut 3mal mit je 163 g n-Hexan für je 2,5 Stunden bei 20 °C extrahiert. Der so entfettete Extraktionsrückstand (15,7 g) wurde getrocknet und anschließend 3mal mit je 200 g Ethanol für je 2,5 Stunden bei 20 °C extrahiert. Die ethanolischen Extrakte wurde vereinigt, das Ethanol wurde bei 50 °C und Drücken von unter 100 mbar abdestilliert. Es wurden 5,89 g eines rot-braunen Pulvers erhalten. Dies entspricht einer Ausbeute von 7,24 Gew.-% bezogen auf das eingesetzte Ausgangsmaterial.

### Beispiel 6b)

81,49 g Erdnusshäutchen (Herkunft China), die einer 4fachen Röstung bei jeweils 95 °C unterzogen wurden, wurden einer Laborstiftmühle mit einem 2 mm Siebeinsatz gefriergemahlen. In einer ersten Extraktionsreihe wurde das Erdnussöl aus den Erdnusshäutchen entfernt, dies geschah mittels einer dreistufigen Kreuzstromextraktion mit n-Hexan: hierzu wurde das Mahlgut 3mal mit je 163 g n-Hexan für je 2,5 Stunden bei 20 °C extrahiert. Der so entfettete Extraktionsrückstand (14,95 g) wurde getrocknet und anschließend 1mal mit 250 g Ethanol für 2,5 Stunden bei 20 °C extrahiert und anschließend 2mal mit je 200 g Ethanol für je 2,5 Stunden bei 20 °C extrahiert. Die ethanolischen Extrakte wurde vereinigt, das Ethanol wurde bei 50 °C und einem Druck von unter 100 mbar abdestilliert. Es wurden 6,54 g eines rot-braunen Pulvers erhalten. Dies entspricht einer Ausbeute von 8,03 Gew.-% bezogen auf das eingesetzte Ausgangsmaterial.

### Beispiel 6c)

81,55 g Erdnusshäutchen (Herkunft Argentinien), die einer 4fachen Röstung bei jeweils 85 °C unterzogen wurden, wurden einer Laborstiftmühle mit einem 2 mm Siebeinsatz gefriergemahlen. In einer ersten Extraktionsreihe wurde das Erdnussöl aus den Erdnusshäutchen entfernt, dies geschah mittels einer dreistufigen Kreuzstromextraktion mit n-Hexan: hierzu wurde das Mahlgut 3mal mit je 163 g n-Hexan für je 2,5 Stunden bei 20 °C extrahiert. Der so entfettete Extraktionsrückstand (13,9 g) wurde getrocknet und anschließend 1mal mit 250 g Ethanol für 2,5 Stunden bei 20 °C extrahiert und danach 2mal mit je 200 g Ethanol für je 2,5 Stunden bei 20 °C extrahiert. Die ethanolischen Extrakte wurde vereinigt, das Ethanol wurde bei 50 °C und einem Druck von unter 100 mbar abdestilliert. Es wurden 6,81 g eines rot-braunen Pulvers erhalten. Dies entspricht einer Ausbeute von 8,35 Gew.-% bezogen auf das eingesetzte Ausgangsmaterial.

### Beispiel 6d)

81,6 g Erdnusshäutchen (Herkunft Argentinien), die einer 4fachen Röstung bei jeweils 115 °C unterzogen wurden, wurden einer Laborstiftmühle mit einem 2 mm Siebeinsatz gefriergemahlen. In einer ersten Extraktionsreihe wurde das Erdnussöl aus den Erdnusshäutchen entfernt, dies geschah mittels einer dreistufigen Kreuzstromextraktion mit n-Hexan: hierzu wurde das Mahlgut 3mal mit je 163 g n-Hexan für je 2,5 Stunden bei 20 °C extrahiert. Der so entfettete Extraktionsrückstand (16,74 g) wurde getrocknet und anschließend 1mal mit 250 g Ethanol für 2,5 Stunden bei 20 °C extrahiert und danach 2mal mit je 200 g Ethanol für je 2,5 Stunden bei 20 °C extrahiert. Die ethanolischen Extrakte wurde vereinigt, das Ethanol wurde bei 50 °C und einem Druck von unter 100 mbar abdestilliert. Es wurden 4,59 g eines rot-braunen Pulvers erhalten. Dies entspricht einer Ausbeute von 5,63 Gew.-% bezogen auf das eingesetzte Ausgangsmaterial.

### Bleichung

Aus dem Extrakt des Beispiels 4 wurden 180 g einer 5 Gew.-%ige ethanolische Lösung hergestellt. Diese wurde mit 36 g Aktivkohle (Norit SA Plus) 5 min. bei 50 °C gerührt. Durch Filtration wurde die Aktivkohle abgetrennt und man erhielt 116 g eines orangefarbenen Filtrates mit einer Konzentration von 2,3 Gew.-%. Nach Entfernung des Ethanols erhielt man ein hellgelbes Pulver.

### 2. Bestimmung der antioxidativen Kapazität der Extrakte

Zur Bestimmung der antioxidativen Kapazität der Erdnusshäutchen Extrakte wurden folgende Methoden eingesetzt.

### Elektronenspinresonanz Spektroskopie (ESR)

Die Methode der Elektronenspinresonanz Spektroskopie beruht auf dem Prinzip der Generierung kurzlebiger Radikale über chemische Radikalgeneratoren, der Stabilisierung und Akkumulierung der Radikale mittels Radikalfänger (Spin-Trapper-Substanzen) und der Detektion der für die einzelnen Radikale typischen ESR-Spektren. Die radikalfangenden Eigenschaften der zu untersuchenden Substanzen werden durch die Abnahme der Signalintensitäten der getrappten Radikale charakterisiert. Im vorliegenden Fall wurde als Radikal Fänger (Spin Trapper) DMPO (5,5-Dimethyl-1-pyrrolin-N-oxid) verwendet. Die Signalintensität des mit Hilfe des DMPO gebildeten stabilen DMPO-Hydroxyadduktes wurde 100 % gesetzt. Die Abnahme dieses Signals in Abhängigkeit von der Konzentration des eingesetzten Pflanzenextraktes, ist ein Nachweis für die Radikal fangenden Eigenschaften der Extrakte.

Der Extrakt aus Beispiel 1 wurde mittels der ESR Spektroskopie untersucht. Bereits in einer Konzentration von 0,01 Gew.-% führte dieser Extrakt zu einer Abnahme des DMPO-Hydroxyaddukt Signals um 79 % auf ein Restsignal von 21 %. Bei einer Konzentration von 0,05 % zeigte sich eine Abnahme des Signals um 89 % und bei einer Konzentration von 0,5 % fand eine völlige Löschung des Signals statt. Als Vergleich diente eine 5 mM α-Tocopherol Lösung, die eine Abnahme des Signals um 65 % bewirkte.

### Bestimmung der antioxidativen Kapazität

Als Maß für die antioxidative Kapazität der Erdnusshäutchen Extrakte wurde ihre Fähigkeit bestimmt, die Lipidperoxidation in Liposomen zu verhindern. Dazu wurde eine 0,2 Gew.-%ige Liposomen Suspension aus Sojalecithin in Phosphatpuffer hergestellt. Die Lipidperoxidation wurde durch Zugabe eines Eisen(II)ascorbat Komplexes (10mM Fe(II)SO₄, 50 mM Ascorbat) initiiert. Die Inkubation wurde bei 37 °C für 45 min. durchgeführt. Als Endprodukt der Lipidperoxidation entsteht Malondialdeyd, welches über eine Farbreaktion mit Thiobarbitursäure quantifiziert wird (photometrische Bestimming des Malondialdehyd-Thiobarbitursäure Komplexes bei 532 nm). Zur Bestimmung der antioxidativen Kapazität der Erdnusshäutchen Extrakte wurden diese in unterschiedlichen Konzentration vor der Zugabe des Eisen(II)ascorbat Komplexes zum Inkubationsansatz gegeben. Ermittelt wurde die Konzentration an Pflanzenextrakt (in µg/ml), bei der die Lipidperoxidation um 50 % verringert ist (= AOP₅₀) im Vergleich zur Bestimmung ohne Zusatz an Pflanzenextrakt. D.h. je geringer die angegebene Konzentration AOP₅₀, desto höher ist die antioxidative Kapazität des untersuchten Erdnussextraktes. Als Referenzsubstanz diente das Antioxidans n-Propylgallat.

**Tab. 1:**

| **Ergebnisse der Bestimmung der antioxidativen Kapazität** | |
|---|---|
| Beispiel | AOP₅₀ [µg/ml] |
| n-Propylgallat (Referenzsubstanz) | 2,9 |
| Extrakt Beispiel 2 | 20,0 |
| Extrakt Beispiel 4 | 4,0 |
| Extrakt Beispiel 6a | 3,71 |
| Extrakt Beispiel 6b | 1,24 |
| Extrakt Beispiel 6c | 3,7 |
| Extrakt Beispiel 6d | 3,57 |

Wie man der Tabelle 1 entnehmen kann, zeigen die Erdnusshäutchen Extrakte eine antioxidative Kapazität, die im Bereich bekannter Antioxidantien (z.B. n-Propylgallat) liegt. Eine vorherige Röstung der Erdnusshäutchen ist dabei ohne entscheidenden Einfluss auf die antioxidative Kapazität wie ein Vergleich des Beispiels 4 (ohne Röstung) mit den Beispielen 6a bis 6d (alle mit Röstung) zeigen.

### 3. Bestimmung der Aktivität gegenüber freien Radikalen

Die optische Dichte des Extraktes lag bei 513 nm. Als erstes Testsubstrat wurde Diphenylpicrylhydrazyl (DPPH) gewählt, ein purpurrot gefärbtes stabiles Radikal, welches durch Inkontaktbringen mit Radikalfängern in sein ungefärbtes Leucoderivat übergeht. Der Farbwechsel kann photometrisch verfolgt werden. Die Meßergebnisse sind in Tabelle 2 zusammengefaßt ("DPPH-Test").. In einem weiteren Test wurde Xanthin Oxidase als Testsystem gewählt. Das Enzym bewirkt bei oxidativem Stress die Umwandlung von Purinbasen, wie z.B. Adenin oder Guanin in Uronsäure, wobei die intermediär gebildeten Sauerstoffradikale durch Reaktion mit Luminol über die Lumineszenz nachgewiesen und quantitativ bestimmt werden können. In Gegenwart von Substanzen mit radikalfangenden Eigenschaften vermindert sich die Lumineszenzausbeute. Der NBT-Wert bestimmt den Gehalt von rotem Tetrazolium-Salz, welches aus NBT durch das Sauerstoffradikal gebildet wird. Die optische Dichte wurde bei 540 nm bestimmt.

Diese Ergebnisse sind in Tabelle 3 zusammengefaßt; wieder ist die Inhibierung in %-absolut angegeben ("Luminol-Test").

**Tab. 2:**

| **Chemische Tests** | | | |
|---|---|---|---|
| EC50 in % | Erdnusshäutchen Extrakt nach Beispiel 1 | Tocopherol | Ascorbinsäure |
| DPPH-Test | 0,0014 % | 0,0067 % | 0,0013 % |

**Tab. 3:**

| **Biochemische Tests** | | | |
|---|---|---|---|
| EC50 in % | Extrakt nach Beispiel 1 | Tocopherol | Ascorbinsäure |
| Luminol | 0,00002 % | Kein Effekt bis zu 1% | 0,0006 % |
| Luminol + Microperoxydase | 0,0053% % | Kein Effekt bis zu 1% | 0,0058% |
| NBT | 0,0074% | Kein Effekt bis zu 1% | 0,5909 % |

Der Extrakt aus Erdnusshäutchen zeigt einen sehr guten Effekt gegen frei Radikale.

### 4. Zellschutzwirkung gegen UVA an in vitro gezüchteten menschlichen Fibroblasten

Hintergrund: UVA-Strahlen dringen bis in die Dermis ein, wo sie zu Oxidationsstreß führen, was durch eine Lipoperoxidation der Zytoplasmamembranen nachgewiesen wird.

Die Lipoperoxide werden zu Malonaldialdehyd (MDA) abgebaut, der viele biologische Moleküle wie Proteine und Nukleinbasen vernetzen wird (Enzymhemmung bzw. Mutagenese).

Methode: Zur Durchführung dieser Tests wurde ein definiertes Kulturmedium mit den Fibroblasten mit fötalem Kälberserum beimpft und der Pflanzenextrakt (in dem definierten Medium mit 10 % Fötalem Kälberserum) 72 Stunden nach dem Beimpfen zugegeben.

Nach 48 stündiger Inkubation bei 37 °C und einem CO₂-Gehalt von 5 % wurde das Kulturmedium durch Saline-Lösung (physiologische NaCI-Lösung) ersetzt und die Fibroblasten wurden mit einer UVA-Dosis bestrahlt (365 nm, 20 J/cm²; Röhren: MAZDA FLUOR TFWN40).

Nach der Beendigung der Bestrahlung wurde der MDA-Spiegel (Malonaldialdehyd-Spiegel) in der überstehenden Natriumchlorid-Lösung quantitativ durch Reaktion mit Thiobarbitursäure bestimmt.

Außerdem wurde der Gehalt an Proteinen nach der Methode von Bradford bestimmt und der Gehalt an GSH untersucht wobei es sich um reduziertes Glutathion handelt. Der Gehalt an GSH wurde bstimmt nach der Methode von Hissin (*Hissin PJ, Hilf R., A fluorometric method for determination of oxydised and reduced glutathione tissues, Anal. Biochem. 74, 214-226, 1976*).

**Tab. 4:**

| **Bestrahlung von humanen Fibroblasten mit UVA (20 J/cm**^{**2**}**) in vitro** | | | |
|---|---|---|---|
| **UVA-Bestrahlung** | **Angaben in % im Vergleich zur Kontrolle** | | |
| **20 J/cm**^{**2**} | **Freigesetzter MDA** | **Zellproteine** | **GSH/Protein** |
| Kontrolle (nicht bestrahlt) | 0 | 100 | 100 |
| ohne Zusatz | 100 | 105 | 72 |
| 0,001 % Extrakt aus Beispiel 1 | 33 | 107 | 72 |
| 0,003 % Extrakt aus Beispiel 1 | 19 | 123 | 70 |
| 0,0003 % Extrakt aus Beispiel 1 | 68 | 109 | 73 |
| 0,0003 % Tocopherol | 19 | 95 | 77 |

Die UVA-Bestrahlung hat zu einem starken Anstieg der Ausschüttung von MDA geführt, während sich der intrazelluläre GSH-Spiegel um ca. 27 % erniedrigte. Zusatz der Erdnusshäutchen-Extrakte vermindert die Menge des freigesetzten MDA und hält den GSH-Spiegel auf hohem Niveau.

### 5. Zellschutzwirkung gegen UVB an in vitro gezüchteten menschlichen Keratinozyten

Hintergrund: UVB-Strahlen lösen durch Aktivierung eines Enzyms, nämlich Phospholipase A2 oder PLA2, das Arachidonsäure aus den Phospholipiden der Plasmamembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet.

Methode: Der Effekt von UVB-Strahlung wurde an Keratinocyten in vitro untersucht indem die Freisetzung des Cytoplasaenzyms LDH (Lactat Dehydrogenase) bestimmt wurde. Dieses Enzym dient als Marker für eine Zellschädigung.

Zur Durchführung der Tests wurde ein definiertes Medium, das fötales Kälberserum enthält, mit den Keratinozyten beimpft und der Pflanzenextrakt (mit Saline-Lösung verdünnt) 72 Stunden nach dem Beimpfen zugegeben.

Die Keratinozyten wurden sodann mit einer UVB-Dosis bestrahlt (50 mJ/cm² - Röhren: DUKE FL40E).

Nach weiterer 1 tägiger Inkubation bei 37 °C und bei 5 % CO₂ wurde der LDH-Gehalt im Überstand bestimmt. Der Gehalt von LDH- (Lactatdehydrogenase) wurde mittels einer Enzymreaktion bestimmt (verwendetes Kit zur Untersuchung des LDH Gehaltes von der Firma Roche). Die Anzahl adhärenter Keratinozyten wird (nach Trypsinbehandlung) mit einem Partikelzählgerät bestimmt.

**Tab. 5:**

| **Zellschutzwirkung von Erdnusshäutchen-Extrakten gegen UVB-Strahlen** | | | |
|---|---|---|---|
| **UVB-Bestrahlung** | **Angaben in % im Vergleich zur Kontrolle** | | |
| **50 J/cm**^{**2**} | **Anzahl Keratinocyten** | **Gehalt an LDH** | **Gehalt an PGE2** |
| Kontrolle (nicht bestrahlt) | 100 | 0 | 0 |
| ohne Zusatz | 24 | 100 | 100 |
| 0,001 % Extrakt aus Beispiel 1 | 85 | 28 | 69 |
| 0,003 % Extrakt aus Beispiel 1 | 126 | 19 | 59 |
| 0,03 % Aspirin | 52 | 46 | 4 |

Die UVB-Bestrahlung hat eine Erhöhung des LDH-Spiegels induziert, während die Zahl der lebensfähigen Keratinozyten um 76 % zurückgegangen ist.

### 6. Inhibierende Effekte auf Proteasen

Bei inflammatorischen Prozessen in der Haut werden von den Makrophagen und von polymorphonuclearen neutrophilen Granulocyten Proteasen wie z.B. die Serin-Protease Elastase oder Matrix-Metallo-Proteasen (MMP) wie Collagenase und eine weitere, zu den MMP gehörende

Elastin abbauende Elastase ausgeschüttet. Des weiteren werden bei älteren Menschen oder nach UV-Strahlung durch die dermalen Fibroblasten interstitial Collagenasen - oder auch MMP-1 genannt - ausgeschüttet.

Diese Proteasen (Collagenase und die unterschiedlichen Elastasen) katalysieren die Fragmentierung und Zerstörung der dermalen Makromoleküle wie Proteoglycan, Collagen und Elastin und führen dadurch zur Alterung der Haut und zu den Effekten der natürlichen Hautalterung nach UV-Strahlung.

### 6a. Inhibierung der Elastase-Aktivität

Serin-Proteasen, wie z.B. eine Art der Elastase, bewirken den Abbau von Elastin, Proteoglycanen und Kollagen und verursachen damit eine Schwächung des Bindegewebes. Im folgenden Test wurden die inhibierenden Eigenschaften des Extraktes nach Beispiel 1 an einem chromogenen synthetischen Substrat (mit Kongorot bezogen von COGER, Frankreich markiert) untersucht. Die Inkubationszeit betrug 30 min bei Raumtemperatur. Die Inhibierung wurde photometrisch bei 520 nm verfolgt, als Positiv-Standard diente α1-Antitrypsin. Die Ergebnisse sind in Tabelle 6 zusammengefasst.

**Tab. 6:**

| **Elastase Inhibierung** | |
|---|---|
| **Testsubstanz** | **EC50 in Gew.-%** |
| Extrakt nach Beispiel 1 | 0,5 |
| α1-Antitrypsin | 0,05 |

Ein Extrakt aus Erdnusshäutchen nach Beispiel 1 führt bei einer Konzentration von 0,5 Gew.-% bereits zu einer 50 prozentigen Inhibierung der Elastase-Aktivität. Der Extrakt zeigt damit eine Aktivität gegen die Serin-Protease Elastase. Diese Aktivität führt zum möglichen Einsatz gegen Effekte der Hautalterung, die in einer erhöhten Aktivität der Serin-Protease Elastase begründet liegen.

### 6b. Inhibierung der MMP-Aktivität

Dermale Fibroblasten älterer Menschen schütten nach Sonnenexposition Collagenasen - auch Matrix Metallo-Protease-1 (MMP-1) - aus. In einem ersten Test wurde Collagenase aus *Clostridium histolyticum* untersucht, welche mit dem Fluoreszenzmarker Fluoresceine von CALBIOCHEM markiert worden war. Die Inkubationszeit betrug 60 min bei Raumtemperatur. Die Hydrolyse von Collagen wurde bestimmt, nach der im Produktblatt des Zulieferers (CALBIOCHEM) beschriebenen Methode. Als Vergleichssubstanz diente Cystein.

**Tab. 7:**

| **Inhibierung der Collagenase** | |
|---|---|
| **Testsubstanz** | **EC50 in Gew.-%** |
| Extrakt nach Beispiel 1 | 0,02 |
| Cystein | 0,24 |

Bereits bei einer Konzentration von 0,02 Gew.-% des Erdnusshäutchen Extraktes ergibt sich eine 50 %ige Inhibierung der Matrix-Metallo-Proteinase Collagenase. Diese Aktivität führt zu dem Einsatz von Erdnusshäutchen-Extrakt gegen Effekte der Hautalterung, die aufgrund eines Abbaus der dermalen Makromoleküle Collagen auftreten.

Im folgenden Test wurden die inhibierenden Eigenschaften des Extraktes nach Beispiel 1 an einem synthetischen Substrat MCA-Pro-Leu-Gly-Leu-DPA-Ala-Arg-NH₂ (Knight et al., 1992, FEBS Letter, 296, S. 263 - 266) und der menschlichen MMP-1 untersucht. Die Inkubationszeit betrug 60 min bei Raumtemperatur. Die Hydrolyse des Substrats wurde fluoreszenzspektrometrisch bei λₑₘ=393 nm (λₑₓ = 328 nm) bestimmt. Als Vergleichsstandard dient der Metallo-Protease-Inhibitor TIMP-1 (natürlich im Gewebe vorkommender Inhibitor für MMP). Die Ergebnisse sind in Tab. 8 dargestellt.

**Tab. 8:**

| **MMP-1-Inhibierung** | | | |
|---|---|---|---|
| **Testsubstanz** | **Konzentration in % (w/v)** | **EC50** | **MMP-1-Inhibierung in %** |
| Kontrolle | - | - | 0 |
| Extrakt nach Beispiel 1 | 0,015 | 0,008 % | 82 |
| | 0,005 | | 33 |
| | 0,0015 | | 9 |
| TIMP-1 | 50 nMol | | 93 |

Auch durch diesen Test zeigt sich eine hohe Aktivität der Extrakte der Erdnusshäutchen, die Matrix-Metallo-Proteinase MMP-1 zu inhibieren.

### 7. Inhibierung der Collagenase-Synthese

Man bedient sich dieses Tests zur Evaluierung der Fähigkeit des Erdnusshäutchen-Extraktes, die toxischen Effekte von UVA-Strahlung an in vitro kultivierten humanen Fibroblasten zu reduzieren. Hierzu wurde sowohl die Menge des ausgeschütteten Enzyms MMP-1 (Matrix-Metallo-Protease) als auch die Menge des natürlichen Enzyminhibitors TIMP-1 bestimmt. Die Zellen wurden mit UVA-Strahlung bestrahlt, da diese bis in die Dermis eindringen und oxidativen Stress induzieren kann, der zur Hautalterung führt.

Die Fibroblasten wurden hierzu in einem genau definierten Medium mit fötalem Kälberserum kultiviert. Der Extrakt nach Beispiel 1 wurde 2 bis 3 Tage nach der Beimpfung zugefügt. Nach einer weiteren Inkubationszeit von einem Tag bei 37 °C und einer CO₂-Konzentration von 5% wurde das Kulturmedium gegen eine Salzlösung ersetzt und die Fibroblasten mit UVA bestrahlt (15 J/cm², Lampe: SOL500, Dr. Höhnle, Filter: H1, Radiometer: Vilbert Lourmat). Nach Beendigung der Bestrahlung wurden die Fibroblasten weitere zwei Tage inkubiert. Der Gehalt an MMP-1 und TIMP-1 im überstehenden Medium wurde mit Hilfe des Untersuchungskits der Fa. Amersham (Kit Nr. RPN2610 und RPN2611) bestimmt. Der Kontrolle wurde kein Extrakt zugesetzt. Tabelle 9 fasstt die erhaltenen Ergebnisse zusammen.

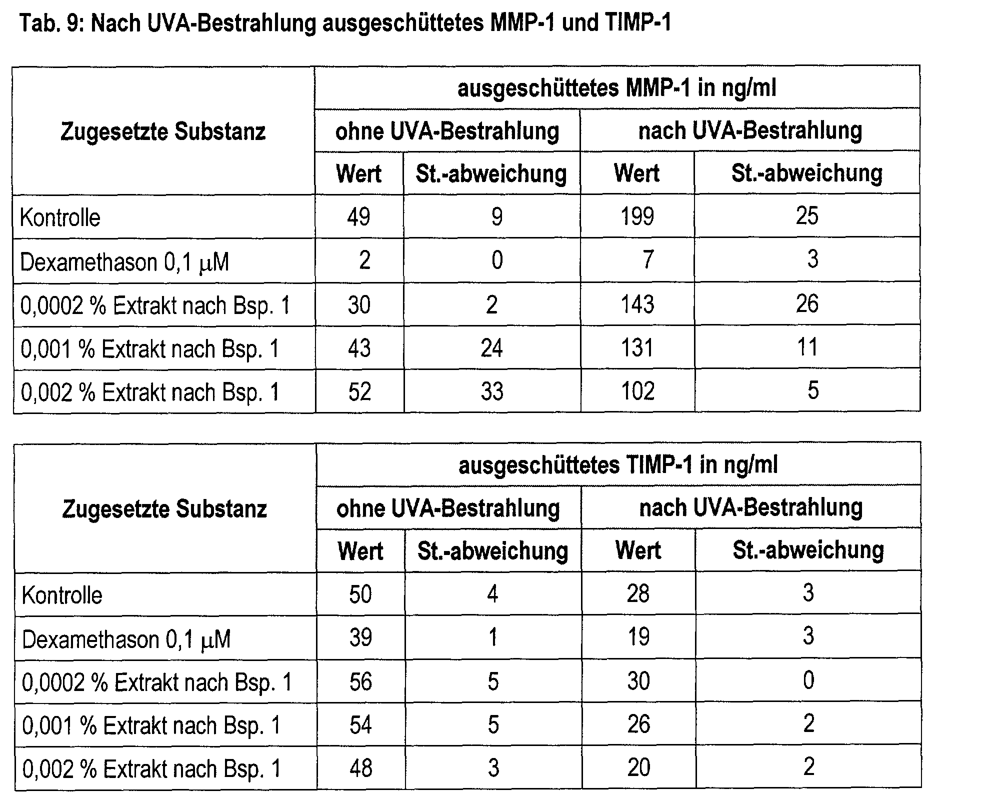

Die Menge des ausgeschütteten MMP-1 nach UVA-Bestrahlung wird deutlich vermindert.

### Beispielrezepturen kosmetischer Mittel mit Erdnusshäutchen Extrakten

Die gemäß Beispiel 1 und 3 gewonnenen Erdnusshäutchen Extrakte wurden in den folgenden erfindungsgemäßen Rezepturen K1 bis K21 sowie 1 bis 35 eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die erfindungsgemäßen Mittel stabil gegen oxidative Zersetzung.

**Tab. 10**

| Softcreme Rezepturen K1 bis K7 | | | | | | | | |
|---|---|---|---|---|---|---|---|---|
| (Alle Angaben in Gew.-% bez. auf das kosmetische Mitteln) | | | | | | | | |
| INCl Bezeichnung | K1 | K2 | K3 | K4 | K5 | K6 | K7 | V1 |
| Glyceryl Stearate (and) Ceteareth-12/20 | 8,0 | 8,0 | 8,0 | 8,0 | 8,0 | 8,0 | 8,0 | 8,0 |
| (and) Cetearyl Alcohol (and) Cetyl | | | | | | | | |
| Palmitate | | | | | | | | |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyl Isononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| **Erdnusshäutchen Extrakt (Beispiel 4)** | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

**Tab. 11**

| **Nachtcremerezepturen K8 bis K14** | | | | | | | | |
|---|---|---|---|---|---|---|---|---|
| (Alle Angaben in Gew.-% bez. auf das kosmetische Mitteln) | | | | | | | | |
| INCI Bezeichnung | K8 | K9 | K10 | K11 | K12 | K13 | K14 | V2 |
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryl Isononanoate | 8,0 | 8,0 | 8,0 | 8,0 | 8,0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| **Erdnusshäutchen Extrakt (Beispiel 4)** | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

**Tab. 12**

| **W/O Bodylotion Rezepturen K15 bis K21** | | | | | | | | |
|---|---|---|---|---|---|---|---|---|
| (Alle Angaben in Gew.-% bez. auf das kosmetische Mitteln) | | | | | | | | |
| INCI-Bezeichnung | K15 | K16 | K17 | K18 | K19 | K20 | K21 | V3 |
| PEG-7 Hydrogenated Castor Oil | 7,0 | 7,0 | 7,0 | 7,0 | 7,0 | 7,0 | 7,0 | 7,0 |
| Decyl Oleate | 7,0 | 7,0 | 7,0 | 7,0 | 7,0 | 7,0 | 7,0 | 7,0 |
| Cetearyl Isononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5,0 | 5,0 | 5,0 |
| MgSO₄*7 H₂O | 1,0 | 1,0 | 1,0 | 1,0 | 1,0 | 1,0 | 1,0 | 1,0 |
| **Erdnusshäutchen Extrakt** | 1,5 | 1,5 | 1,5 | 1,5 | 1,5 | 1,5 | 1,5 | - |
| **(Beispiel 4)** | | | | | | | | |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

| | | | | | | | | |
|---|---|---|---|---|---|---|---|---|
| ¹⁾ Desoxyribonucleinsäure: Molekuargewicht ca. 70000, Reinheit (bestimmt durch spektro-photometrische Messung der Absorption bei 260 nm sowie 280 nm): mindestens 1,7. | | | | | | | | |

**Tab. 13**

| **Rezepturen** | | | | | | |
|---|---|---|---|---|---|---|
| **Kosmetische Zubereitungen Conditioner (Wasser, Konservierungsmittel ad 100 Gew.-%)** | | | | | | |
| **Zusammensetzung (INCl)** | **1 Gew.- %** | **2 Gew.- %** | **3 Gew.- %** | **4 Gew.- %** | **5 Gew.- %** | **6 Gew.- %** |
| **Dehyquart® A** Cetrimonium Chloride | 4,0 | 4,0 | | | 3,0 | |
| **Dehyquart L® 80** Dicocoylmethylethoxymonium Methosulfate (and) Propylenglycol | | | 1,2 | 1,2 | | 1,0 |
| **Eumulgin® B2** Ceteareth-20 | 0,8 | | - | 0,8 | - | 1,0 |
| **Eumulgin® VL 75** Lauryl Glucoside (and) Polyglyceryl-2 Polyhydroxystearate (and) Glycerin | - | 2,0 | 2,0 | - | 0.8 | - |
| **Lanette® O** Cetearyl Alcohol | 3,0 | 3,0 | 3,0 | 3,0 | 3,0 | 3,0 |
| **Cutina® GMS** Glyceryl Stearate | - | 0,5 | - | 0,5 | - | 1,0 |
| **Lamesoft® PO 65** Coco-Glucosid (and) Glyceryl Oleate | | - | 3,0 | - | - | 3,0 |
| **Cetiol® J 600** Oleyl Erucate | - | 0,5 | - | 1,0 | - | 1,0 |
| **Eutanol® G** Octyldodecanol | - | - | 1,0 | - | - | 1,0 |
| **Nutrilan® Keratin W** Hydrolyzed Keratin | 5,0 | - | - | 2,0 | - | - |
| **Generol® 122 N** Soja Sterol | - | - | - | - | 1,0 | 1,0 |
| **Erdnusshäutchen Extrakt gemäß Beispiel 1** | 1,0 | 1,0 | 1,0 | 1,0 | 1,0 | 1,0 |
| **Copherol® 1250** Tocopheryl Acetate | - | - | 0,1 | 0,1 | - | - |
| (1-4) Haarspülung, (5-6) Haarkur | | | | | | |

**Tab. 13**

| **Rezepturen** | | | | |
|---|---|---|---|---|
| **Kosmetische Zubereitungen Conditioner (Wasser, Konservierungsmittel ad 100 Gew.-%)** | | | | |
| **Zusammensetzung (INCl)** | **7 Gew.- %** | **8 Gew.- %** | **9 Gew.- %** | **10 Gew.- %** |
| **Texapon® NSO** Sodium Laureth Sulfate | 38,0 | 38,0 | 25,0 | - |
| **Texapon® SB 3** Disodium Laureth Sulfosuccinate | - | - | 10,0 | - |
| **Plantacare® 818** Coco Glucosides | 7,0 | 7,0 | 6,0 | - |
| **Plantacare® PS 10** Sodium Laureth Sulfate (and) Coco Glucosides | - | - | - | 20,0 |
| **Dehyton® PK 45** Cocamidopropyl Betaine | - | - | 10,0 | |
| **Lamesoft® PO 65** Coco-Glucosid (and) Glyceryl Oleate | 3,0 | | | 4,0 |
| **Lamesoft® LMG** Glyceryl Laurate (and) Potassium Cocoyl Hydrolyzed Collagen | - | 5,0 | - | |
| **Euperlan® PK 3000 AM** Glycol Distearate (and) Laureth-4 (and) Cocamidopropyl Betaine | - | 3,0 | 5,0 | 5,0 |
| **Erdnusshäutchen Extrakt gemäß Beispiel 1** | 1,0 | 1,0 | 1,0 | 1,0 |
| **Arlypon® F** Laureth-2 | 3,0 | 3,0 | 1,0 | - |
| **Sodium Chloride** | - | 1,5 | - | 1,5 |
| (7-8) Duschbad, (9) Duschgel, (10) Waschlotion | | | | |

**Tab. 13**

| **Kosmetische Zubereitungen Duschbad "Two in One" (Wasser, Konservierungsmittel ad 100 Gew.-%) -** Fortsetzung | | | | |
|---|---|---|---|---|
| **Zusammensetzung (INCl)** | **11** | **12** | **13** | **14** |
| **Texapon® NSO** Sodium Laureth Sulfate | 30,0 | 25,0 | | 25,0 |
| **Plantacare® 818** Coco Glucosides | | | | 8,0 |
| **Plantacare® 2000** Decyl Glucoside | | 8,0 | | |
| **Plantacare® PS 10** Sodium Laureth Sulfate (and) Coco Glucosides | | | 20,0 | |
| **Dehyton® PK 45** Cocamidopropyl Betaine | | 10,0 | 10,0 | |
| **Lamesoft® PO 65** Coco-Glucosid (and) Glyceryl Oleate | 5,0 | | | |
| **Lamesoft® LMG** Glyceryl Laurate (and) Potassium Cocoyl Hydrolyzed Collagen | | 5,0 | 5,0 | |
| **Gluadin® WQ** Laurdimonium Hydroxapropyll Hydrolyzed Wheat Protein | 3,0 | | | |
| **Gluadin® WK** Sodium Cocoyl Hydrolyzed Wheat Protein | | | | |
| **Euperlan® PK 3000 AM** Glycol Distearate (and) Laureth-4 (and) Cocamidopropyl Betaine | 5,0 | 3,0 | 4,0 | - |
| **Panthenol** | 0,5 | - | - | 0,5 |
| **Erdnusshäutchenextrakt gemäß Beispiel 1** | 1,0 | 1,0 | 1,0 | 1,0 |
| **Arlypon® F** Laureth-2 | 2,6 | 1,6 | - | 1,0 |
| **Sodium Chloride** | - | - - | | - |

**Tab. 13**

| **Kosmetische Zubereitungen Shampoo (Wasser, Konservierungsmittel ad 100 Gew.-%) - Fortsetzung** | | | | | | |
|---|---|---|---|---|---|---|
| **Zusammensetzung (INCl)** | **15** | **16** | **17** | **18** | **19** | **20** |
| **Texapon® NSO** Sodium Laureth Sulfate | 30,0 | | | 30,0 | 25,0 | |
| **Texapon® K 14 S** Sodium Myreth Sulfate | | 30,0 | | | | 30,0 |
| **Texapon® SB 3** Disodium Laureth Sulfosuccinate | | 10,0 | | | | |
| **Plantacare® 818** Coco Glucosides | 4,0 | | | | | |
| **Plantacare® 2000** Decyl Glucoside | | 4,0 | | | | |
| **Plantacare® PS 10** Sodium Laureth Sulfate (and) Coco Glucosides | | | 20,0 | | | |
| **Dehyton® PK 45** Cocamidopropyl Betaine | 5,0 | | | 10,0 | | 10,0 |
| **Gluadin® WK** Sodium Cocoyl Hydrolyzed Wheat Protein | | | | | 8,0 | |
| **Lamesoft® PO 65** Coco-Glucosid (and) Glyceryl Oleate | - | - | - | - | 2,0 | 2,0 |
| **Nutrilan® Keratin W** Hydrolyzed Keratin | 5,0 | - | - | - | | - |
| **Gluadin® W 40** Hydrolyzed Wheat Protein | - | 2,0 | - | 2,0 | - | - |
| **Euperlan® PK 3000 AM** Glycol Distearate (and) Laureth-4 (and) Cocamidopropyl Betaine | - | - | - | 3,0 | 3,0 | - |
| **Panthenol** | - | - | - | - | - | 0,2 |
| **Erdnusshäutchenextrakt gemäß Beispiel 1** | 1,0 | 1,0 | 1,0 | 1,0 | 1,0 | 1,0 |
| **Arlypon® F** Laureth-2 | 1,5 | - | - | - | - | - |
| **Sodium Chloride** | - | 1,6 | 2,0 | 2,2 | - | 3,0 |

**Tab. 13**

| **Kosmetische Zubereitungen Schaumbad (Wasser, Konservierungsmittel ad 100 Gew.-%) - Fortsetzung 2** | | | | | |
|---|---|---|---|---|---|
| **Zusammensetzung** Bezeichnung nach INCI | **21** | **22** | **23** | **24** | **25** |
| **Texapon® NSO** Sodium Laureth Sulfate | - | 30,0 | 30,0 | - | 25,0 |
| **Plantacare® 818** Coco Glucosides | - | 10,0 | - | - | 20,0 |
| **Plantacare® PS 10** Sodium Laureth Sulfate (and) Coco Glucosides | 22,0 | - | 5,0 | 22,0 | - |
| **Dehyton® RK 45** Cocamidopropyl Betaine | 15,0 | 10,0 | 15,0 | 15,0 | 20,0 |
| **Monomuls® 90-O 18** Glyceryl Oleate | 0,5 | | | | |
| **Lamesoft® PO 65** Coco-Glucosid (and) Glyceryl Oleate | | 3,0 | | 3,0 | |
| **Cetiol® HE** PEG-7 Glyceryl Cocoate | | | 2,0 | | 2,0 |
| **Nutrilan® I-50** Hydrolyzed Collagen | 5,0 | | | | |
| **Gluadin® W 40** Hydrolyzed Wheat Gluten | | 5,0 | | 5,0 | |
| **Gluadin® WK** Sodium Cocoyl Hydrolyzed Wheat Protein | | | | 7,0 | |
| **Euperlan® PK 3000 AM** Glycol Distearate (and) Laureth-4 (and) Cocamidopropyl Betaine | 5,0 | - | - | 5,0 | - |
| **Arlypon® F** Laureth-2 | | | 1,0 | | |
| **Sodium Chloride** | 1,0 | | 1,0 | | 2,0 |
| **Erdnusshäutchenextrakt gemäß Beispiel 1** | 1,0 | 1,0 | 1,0 | 1,0 | 1,0 |

**Tab. 13**

| **Kosmetische Zubereitungen (Wasser, Konservierungsmittel ad 100 Gew.-%) - Fortsetzung 3** | | | | | | | | | | |
|---|---|---|---|---|---|---|---|---|---|---|
| **Zusammensetzung (INCl)** | **26** | **27** | **28** | **29** | **30** | **31** | **32** | **33** | **34** | **35** |
| **Dehymuls® PGPH** Polyglyceryl-2 Dipolyhydroxystearate | 4,0 | 3,0 | - | 5,0 | - | - | - | - | - | - |
| **Lameform® TGI** Polyglyceryl-3 Diisostearate | 2,0 | 1,0 | - | - | - | - | - | - | - | - |
| **Emulgade® PL 68/50** Cetearyl Glucoside (and) Cetearyl Alcohol | - | - | - | - | 4,0 | - | - | - | 3,0 | - |
| **Eumulgin® B2** Ceteareth-20 | - | - | - | - | - | - | - | 2,0 | - | - |
| **Tegocare® PS** Polyglyceryl-3 Methylglucose Distearate | - | - | 3,0 | - | - | - | 4,0 | - | - | - |
| **Eumulgin VL 75** Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2,0 | 5,0 | 2,0 | - | - | - | - | - | - |
| **Cutina® GMS** Glyceryl Stearate | - | - | - | - | - | 2,0 | 4,0 | - | - | 4,0 |
| **Lanette® O** Cetearyl Alcohol | - | - | 2,0 | - | 2,0 | 4,0 | 2,0 | 4,0 | 4,0 | 1,0 |
| **Antaron® V216** PVP / Hexadecene Copolymer | - | - | - | - | - | 3,0 | - | - | - | 2,0 |
| **Myritol® 818** Cocoglycerides | 5,0 | - | 10,0 | - | 8,0 | 6,0 | 6,0 | - | 5,0 | 5,0 |
| **Finsolv® TN** C12/15 Alkyl Benzoate | - | 6,0 | - | 2,0 | - | - | 3,0 | - | - | 2,0 |
| **Cetiol® J 600** Oleyl Erucate | 7,0 | 4,0 | 3,0 | 5,0 | 4,0 | 3,0 | 3,0 | - | 5,0 | 4,0 |
| **Cetiol® OE** Dicaprylyl Ether | 3,0 | - | 6,0 | 8,0 | 6,0 | 5,0 | 4,0 | 3,0 | 4,0 | 6,0 |
| **Mineral Oil** | - | 4,0 | - | 4,0 | - | 2,0 | - | 1,0 | - | - |
| **Cetiol® PGL** Hexadecanol (and) Hexyldecyl Laurate | - | 7,0 | 3,0 | 7,0 | 4,0 | - | - | - | 1,0 | - |
| **Panthenol / Bisabolol** | 1,2 | 1,2 | 1,2 | 1,2 | 1,2 | 1,2 | 1,2 | 1,2 | 1,2 | 1,2 |
| **Erdnusshäutchenextrakt gemäß Beispiel** 1 | 1,0 | 1,0 | 1,0 | 1,0 | 1,0 | 1,0 | 1,0 | 1,0 | 1,0 | 1,0 |
| **Copherol® F 1300** Tocopherol / Tocopheyl Acetate | 0,5 | 1,0 | 1,0 | 2,0 | 1,0 | 1,0 | 1,0 | 2,0 | 0,5 | 2,0 |
| **Neo Heliopan® Hydro** Sodium Phenylbenzimidazole Sulfonate | 3,0 | - | - | 3,0 | - | - | 2,0 | - | 2,0 | - |
| **Neo Heliopan® 303** Octocrylene | - | 5,0 | - | - | - | 4,0 | 5,0 | - | - | 10,0 |
| **Neo Heliopan® BB** Benzophenone-3 | 1,5 | - | - | 2,0 | 1,5 | - | - | - | 2,0 | - |
| **Neo Heliopan® E 1000** Isoamyl p-Methoxycinnamate | 5,0 | - | 4,0 | - | 2,0 | 2,0 | 4,0 | 10,0 | - | - |
| **Neo Heliopan® AV** Octyl Methoxycinnamate | 4,0 | - | 4,0 | 3,0 | 2,0 | 3,0 | 4,0 | - | 10,0 | 2,0 |
| **Uvinul® T 150** Octyl Triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |
| (26) W/O-Sonnenschutzcreme, (27-29) W/O-Sonnenschutzlotion, (30, 33, 35) O/W-Sonnenschutzlotion (31, 32, 34) O/W-Sonnenschutzcreme | | | | | | | | | | |

## Patentansprüche

1. Kosmetische und/oder pharmazeutische Mittel, enthaltend einen Extrakt aus Samenhäutchen von Arachis hypogaea L. (Erdnuss).

2. Mittel nach Anspruch 1, **dadurch gekennzeichnet, dass** sie Flavonderivate enthalten, die ausgewählt sind aus der Gruppe, die gebildet wird von Luteolin, procyanidolischen Oligomeren, Procyanidin B1, Proanthocyanidinen, Proanthioanthocyanidin A2.

3. Mittel nach Anspruch 1 und/oder 2, **dadurch gekennzeichnet, dass** sie 0,01 bis 20 Gew.-%-bezogen auf das Mittel- des Extraktes enthalten.

4. Verwendung eines Extraktes aus Samenhäutchen von Arachis hypogaea L. als Pflegemittel für Haut und/oder Haare.

5. Verwendung eines Extraktes aus Samenhäutchen von Arachis hypogeae L als Antioxidans in kosmetischen Mitteln.

6. Verwendung eines Extraktes aus Samenhäutchen von Arachis hypogaea L. gegen freie Radikale.

7. Verwendung eines Extraktes aus Samenhäutchen von Arachis hypogaea L. als Sonnenschutzmittel, insbesondere gegen UVA- und/oder gegen UVB-Strahlung.

8. Verwendung eines Extraktes aus Samenhäutchen von Arachis hypogaea L. als anti-inflammatorisches Mittel, insbesondere als anti-rosacea Mittel.

9. Verwendung eines Extraktes aus Samenhäutchen von Arachis hypogaea L. gegen die Hautalterung.

10. Verwendung eines Extraktes aus Samenhäutchen von Arachis hypogaea L. als Protease-inhibierendes Mittel, insbesondere als Matrix-Metallo-Proteinase (MMP)- und/oder Collagenaseund/oder Elastase-inhibierendes Mittel.

11. Verwendung eines Extraktes aus Samenhäutchen von Arachis hypogaea L. als Stimulator der Tissue-inhibitor of Metallo Protease (TIMP).
